# EUROPÄISCHE PATENTSCHRIFT

(11) **EP 1 766 401 B1**
(45) Veröffentlichungstag und Bekanntmachung des Hinweises auf die Patenterteilung: **25.03.2009**
(21) Anmeldenummer: 05772506.1
(22) Anmeldetag: 12.07.2005
(51) Int. Cl.: G01N 33/543, C12Q 1/68

(54) **VERFAHREN ZUM EINFACHEN UND SCHNELLEN NACHWEIS VON ZELLEN UND BIOMOLEKÜLEN MIT HILFE PARAMAGNETISCHER PARTIKEL**
METHOD FOR THE SIMPLE AND RAPID DETECTION OF CELLS AND BIOMOLECULES BY MEANS OF PARAMAGNETIC PARTICLES
PROCEDE DE DETECTION SIMPLE ET RAPIDE DE CELLULES ET DE BIOMOLECULES A L'AIDE DE PARTICULES PARAMAGNETIQUES

(30) Priorität: 12.07.2004 DE 102004033811
(43) Veröffentlichungstag der Anmeldung: 28.03.2007
(73) Patentinhaber: Kiesewetter, Holger, 13465 Berlin (DE); Salama, Abdulgabar, 13467 Berlin (DE)
(72) Erfinder: KIESEWETTER, Holger, 13465 Berlin (DE); SALAMA, Abdulgabar, 13467 Berlin (DE); ADAM, John, (CH)
(74) Vertreter: Luderschmidt, Wolfgang
(86) Internationale Anmeldenummer: PCT/EP2005/007571
(87) Internationale Veröffentlichungsnummer: WO 2006/005593

(56) Entgegenhaltungen:
- DE-A1- 4 124 778
- DE-A1-3102004 005 19
- US-A- 5 145 784
- US-A- 5 283 079
- YAN K -T ET AL: "Development of an immunomagnetic antigen capture system for detecting leptospire in bovine urine" RESEARCH IN VETERINARY SCIENCE, Bd. 64, Nr. 2, März 1998 (1998-03), Seiten 119-124, XP002351608 ISSN: 0034-5288

## Beschreibung

Die vorliegende Erfindung betrifft ein einfaches und schnell durchführbares Verfahren zum Nachweis von Zellen und Biomolekülen mit Hilfe paramagnetischer Partikel (Beads). Die zu untersuchende Probe wird mit paramagnetischen Beads (kommerziell erhältlich von verschiedenen Herstellern), die mit monospezifischen Antikörpern oder Antigenen (Detektionsmoleküle) beladen sind, versetzt und gemischt. Die Probe mit den Beads wird dann in ein starkes Magnetfeld gelegt. Durch das Magnetfeld werden die Beads festgehalten, und der Rest der Probe wird komplett entfernt oder abgesaugt. Nach Entfernung des Magnetfeldes werden die Partikel suspendiert. Eine Verklumpung der Partikel spricht dann für das Vorliegen einer spezifischen Reaktion bzw. für eine spezifische Isolierung von Ziel-Zellen bzw. Ziel-Biomolekülen, da diese mit den spezifischen Detektionsmolekülen reagieren und zusammen mit den Beads eine sichtbare Vernetzung (Agglutination) darstellen. Weiterhin kann das Vorliegen einer spezifischen Reaktion auch durch photometrische Bestimmung der Bindung des spezifischen Biomoleküls nachgewiesen werden. In diesem Fall werden bevorzugt markierte Beads oder Antikörper eingesetzt.

Charakteristisch an dem Verfahren ist die extrem hohe Sensitivität, die einfache Handhabung, die schnelle Testung und Beurteilung zu jeder Zeit und an jedem Ort. Die Reaktion kann durch die Beigabe von anderen, nicht paramagnetischen und kleineren Beads, die auch mit spezifischen Detektionsmolekülen beladen sind, sichtbar oder massiv verstärkt werden. Eine weitere Detektion oder Verstärkung der Reaktion ist durch die Testung im Gelkartensystem (Partikelagglutination-Testsystem) möglich oder durch photometrische Messung insbesondere bei farbigen oder mit Europium markeirten Beads.

Der Nachweis von Zellen.und Biomolekülen ist.in zahlreichen Bereichen notwendig, wie z.B. der Nachweis von kindlichen Zellen im maternalen Kreislauf, die Früherkennung von Tumorzellen im Kreislauf betroffener Patienten, der Nachweis von Tumorzellen in autologen Stammzellpräparaten, der Nachweis von Proteinen (Moleküle in der Körperflüssigkeit oder dem Gewebe), der Nachweis von Bakterien oder Keimen in Proben oder Nahrungsmitteln, der Nachweis von Toxinen (Biowaffen) usw. Die bisher verwendeten empfindlichen Techniken sind relativ aufwändig und können nur in Speziallaboratorien durchgeführt werden, wie z.B. die Flowzytometrie (FACS), Polymerase-Kettenreaktionen (PCR) und die lmmunhistochemie [1-16]. Paramagnetische und nichtparamagnetische Mikro- und Nanobeads werden von verschiedenen Herstellern (Dynal, Miltenyi, Biotec u.a.) angeboten, und die Aufreinigung von Zellen und Biomolekülen mit Hilfe paramagnetischer Beads ist ein bekanntes Verfahren [DE 101 11 520 A1;
WO 02/090565 A2; DE 101 37 665 A1; US 2002/0072129 A1; EP 0 855 441 A2; US 2003/0175691 A1; 4,649,116; US 2003/0032028 A1; US 2004/0005718 A1]. Mit Hilfe solcher paramagnetischen Beads können aus einer Lösung bestimmte Zeilen oder Moleküle isoliert und charakterisiert werden.

DE4124778 beschreibt ein Verfahren zur Analyse von Agglutinationsreaktionen unter Verwendung von magnetischen Partikeln und Mikrosäulen.

In einem typischen Protokoll wird eine Suspension von Magnetpartikeln mit einer Probe versetzt, die bestimmte Zellen oder Moleküle enthält. Anschließend wird ein Magnetfeld angelegt, so dass die Partikel mit den Ziel-Zellen bzw. Ziel-Molekülen durch das Magnetfeld an einer Wand des Gefäßes festgehalten werden. Der Überstand wird verworfen, und die Partikel werden noch mindestens einmal gewaschen. Danach gibt man einen geeigneten Puffer zur Trennung der isolierten Zellen bzw. Biomoleküle von den Beads zu. Die Trennung geschieht durch Zentrifugation oder durch das Anlegen eines Magnetfeldes. Die getrennten Zielmoleküle werden mit den oben erwähnten, relativ komplizierten Verfahren wie z.B. FACS, PCR oder immunhistochemische Methoden nachgewiesen. Die Nachteile aller bisher beschriebenen Verfahren in diesem Zusammenhang sind:
1. die immer vorhandene Notwendigkeit einer Abtrennung der ZielMoleküle von den Beads,
2. der dadurch bedingte Verlust von Ziel-Molekülen (durch das Abtrennen),
3. der Arbeitsaufwand,
4. der Zeitaufwand,
5. der Kostenaufwand,
6. die meist komplizierte Durchführbarkeit, sowie
7. die Notwendigkeit von Spezialgeräten.

Aufgabe der vorliegenden Erfindung war es daher, ein Verfahren zu entwickeln, mit dem die Nachteile des vorstehend diskutierten Stands der Technik überwunden werden. Insbesondere sollte das Verfahren schnell und einfach in der Durchführung sein und dabei eine hohe Sensitivität ermöglichen.

Unter einem ersten Gesichtspunkt betrifft die Erfindung daher ein Verfahren zum Nachweis von Zellen oder Biomolekülen in einer Probe, wobei man:
(a) paramagnetische Beads mit einem gegen ein Merkmal des nachzuweisenden Biomoleküls oder der nachzuweisenden Zelle gerichteten Detektionsmolekül (bevorzugt einem spezifischen Antikörper oder einem Antigen) beschichtet,
(b) die zu untersuchende Probe mit den beschichteten Beads in Kontakt bringt,
(c) die Probe von den nun beladenen Beads entfernt,
(d) die beladenen und von der Probe entfernten Mikrobeads in Suspension bringt
(e) die in (d) erhaltene Mikrobeads-Suspension im Gelkartensystem untersucht, und
(f) anhand des Vorliegens einer spezifischen Reaktion das Vorhandensein des nachzuweisenden Biomoleküls bzw. der nachzuweisenden Zelle beurteilt.

Dieses Verfahren wird mit Vorteil so ausgeführt, dass in Stufe a) und/oder b) zusätzlich zu den Mikrobeads Nanobeads oder Antikörper, die mit einem zweiten Detektionsmolekül beschichtet sind, welches entweder gegen das gleiche Merkmal oder gegen ein weiteres Merkmal auf der Suchzelle / dem Biomolekül gerichtet ist, in der Bindungsreaktion als sogenannter Agglutinationsverstärker (bzw. Verstärkungsbeads) mitgeführt werden.

Solche Agglutinationsverstärker können in einem weiteren bevorzugten Ausführungsbeispiel der vorliegenden Erfindung alternativ hierzu auch erst nach Stufe (c) des erfindungsgemäßen Verfahrens zugesetzt werden. Die Testung wird im Gelkartensystem (Partikelagglutination-Testsystem) durchgeführt.

Erfindungsgemäß weisen Nanopartikel bevorzugt eine Größe von 50nm bis 0,5 µm, bevorzugt etwa 100 nm auf.

Besonders bevorzugt sind die Nanopartikel und/oder Mikrobeads farbig, was eine photometrische Auswertung ermöglicht.

Ein erfindungsgemäß besonders bevorzugtes System für den Nachweis sind Europium enthaltende Beads, insbesondere Nanobeads oder auch Antikörper. Mittels der zeitverzögerten Floureszens lassen sich diese Beads und damit die gebildeten Agglutinate hochempfindlich nachweisen. Europium-Nanopartikel können beispielsweise von der Firma Seradyn, Indianapolis, USA bezogen werden. Diese Partikel sind mit Europium-Chelaten "aufgesaugt", so dass die Oberfläche frei für Kopplungsreaktionen bleibt und die Europium-Chelate nicht auslaufen. Europium-Chelat: tris-(naphtyltrifluorobutandion)-Eu. Wenn diese Partikel mit UV-Licht (333 nm Maximum) angeregt werden, emittieren sie eine Lichtausstrahlung bei 613 nm mit einer Zeitdauer von ungefähr 0,5 Millisekunden, was 10 000fach bis 100 000fach länger als die Emissionsdauer der meisten Fluorophore ist. Diese extrem lange Emissionsdauer und die große Stokes-Verschiebung (Differenz zwischen Emissions- und Erregungswellenlänge) erlaubt ihre Verwendung in den auf zeitverzögerter Fluoreszenz basierten Tests. Jedes Partikel enthält >30 000 Europium-Atome, eingeschlossen in tris-Naphtyltrifluorobutandion (ein Diketon). Für die 100 nm großen Partikel ist der s.g. Quantumertrag (quantum yield, engl.) equivalent zu circa 3 000 Molekülen Fluorescein (einer der meist verwendeten Fluorophore). Das Phycobiliprotein (die wahrscheinlich am stärksten fluoreszierende bekannte Verbindung) hat zum Vergleich einen Quantengewinn, der dem von ca. 30 Fluorescein Molekülen entspricht. Da ein 100 nm Partikel einen 10 mal größeren Durchmesser im Vergleich zu dem Phycobiliprotein und ein 1 000 mal größeres VolumenlMasse Verhältnis hat, weisen diese Beads eine 100 mal höhere Fluoreszenz auf molarer Basis auf, als das Phycobiliprotein.

Erfindungsgemäße Mikropartikel weisen eine Größe zwischen 0,1 bis 5 µm, bevorzugt 2,5 bis 3 µm auf.

Mit Vorteil wird die Beurteilung des Vorliegens einer Agglutination visuell oder photometrisch durchgeführt.

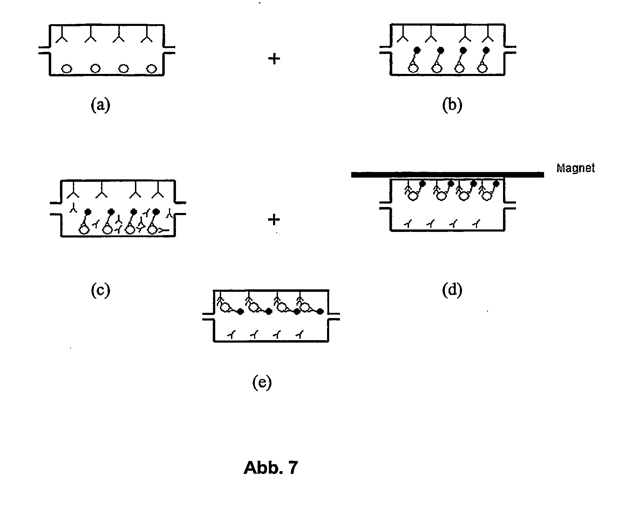

Optional kann (können) auch zwischen Stufe (c) und (d) einer oder mehrere Waschschritt(e) durchgeführt werden. Auf diese(n) Waschschritt(e) kann mit Vorteil verzichtet werden, wenn die nachzuweisenden Biomoleküle Antikörper sind, und ein Vorgehen entsprechend Abb. 7 gewählt wird.

Biomoleküle sind insbesondere Antigene und Antikörper, prinzipiell lassen sich aber alle dem Fachmann geläufigen Biomoleküle wie Proteine, Nukleinsäuren und weitere so nachweisen. Insbesondere kann es sich um solche Antikörper handeln, wie sie beispielsweise bei der Blutgruppenbestimmung nachgewiesen werden.

Detektionsmoleküle hängen naturgemäß von dem spezifischen nachzuweisenden Biomolekül ab. Bevorzugt handelt es sich bei den Detektionsmolekülen um spezifische Antikörper oder Antigene, es kann sich jedoch auch um andere bindende Moleküle handeln, wie beispielsweise um Nukleinsäuren usw.

Mit dem Ausdruck "Merkmal des nachzuweisenden Biomoleküls oder der nachzuweisenden Zelle (alternative Bezeichnung: Zielzelle, kurz ZZ)" ist erfindungsgemäß z.B. eine antigene Determinante (Epitop) des Biomoleküls bzw. der nachzuweisenden Zelle gemeint. Es kann sich jedoch z.B. auch um eine komplementäre Nukleinsäure, oder weitere bindende Prinzipien handeln.

Mit dem erfindungsgemäßen Verfahren werden die Nachteile des Stands der Technik überwunden, indem die isolierten Zellen bzw. Biomoleküle nicht von den Partikeln getrennt werden, sondern die Reaktion, vorzugsweise eine Agglutination, zwischen den Komponenten visuell oder photometrisch direkt beurteilt wird (siehe Abb. 1). Tritt eine Agglutination oder eine spezifische Bindungsreaktion, die nicht zum Agglutinieren der Partikel führt, auf, so handelt es sich um eine spezifische Reaktion, die Probe gilt als positiv. Die Empfindlichkeit kann durch die Mengenbeimischung und durch die Verwendung von kommerziell erhältlichen Gelkarten für den Nachweis erythrozytärer Agglutination oder Partikelagglutination (z.B. das ID-Kartensystem der Fa. DiaMed) gesteigert werden (Abb. 2). Eine weitere Verstärkung der Sichtbarmachung der Reaktionen kann durch eine Beimischung mit kleineren Beads (Nanobeads) (50 nm - 0,5 µm) gesteigert werden (Abb. 3). Diese Verstärkungsbeads werden mit Antikörpern oder Antigenen, die gegen das gleiche Biomolekül bzw. die gleiche Suchzelle gerichtet sind, vorbeladen. Je nach Messverfahren können farblose oder gefärbte Verstärkungsbeads verwendet werden. Im letzten Fall können die Reaktionen auch photometrisch gemessen werden. Die Verstärkungsbeads können sowohl gleichzeitig (Abb. 3) mit den paramagnetischen Beads mit der Ausgangsprobe versetzt werden oder nach Isolierung und Reinigung (Abb. 4) des Zielmoleküls dazu gegeben werden. Anstelle von Verstärkerbeads können auch markierte Antikörper verwendet werden, wobei die im Stand der Technik bekannten Markierungsmethoden verwendet werden können. Eine nicht-radioaktive Markierung ist hierbei bevorzugt (Abb. 5 und Abb. 6). Die Reaktionen können visuell, automatisch oder photometrisch abgelesen werden. Darüber hinaus eignet sich die Methode nicht nur für den Nachweis von antigenen Biomolekülen, sondern auch von Antikörpern. Hier kann auf den Waschvorgang, der für die Entfernung von freien spezifischen oder unspezifischen nicht gebundenen Antikörpern dient, verzichtet werden (Abb. 7). Waschen ist jedoch auch möglich (Abb. 8).

Unter einem bevorzugten Gesichtspunkt betrifft die vorliegende Methode daher ein Verfahren zum Nachweis spezifischer Antikörper in einer Probe, wobei man zusätzlich zur oben genannten Stufe a) in einer zusätzlichen Stufe a1) ein Antigen, gegen das der nachzuweisende Antikörper gerichtet ist, auf einem Trägermittel (I) vorlegt, ohne es an das Trägermittel fest zu binden, in einer zusätzlichen Stufe a2) ein weiteres Trägermittel (II) mit einem anti-Spezies X Antikörper beschichtet, wobei Spezies X die Spezies ist, aus der der nachzuweisende Antikörper stammt, und wobei die Trägermittel so angeordnet sind, dass die zu untersuchende Probe in Stufe b) zunächst nur mit dem Trägermittel (I) in Kontakt treten kann, in einer zusätzlichen Stufe a3) die beschichteten Beads aus Stufe a) mit dem Antigen aus Stufe a1) in Kontakt bringt, und der Magnet in Stufe c) so angelegt wird, dass bedingt durch die magnetische Kraft die Konglomerate aus beschichteten Beads, Antigen und nachzuweisendem Antikörper zu dem mit anti-X beschichteten Trägermittel bewegt werden, wobei das Vorhandensein des nachzuweisenden Antikörpers in der Probe durch die Bildung spezifischer Agglutinate bzw. spezifischer Bindungsreaktionen zwischen diesem nachzuweisenden Antikörper und dem anti-X Antikörper bestimmt wird. Dieses Vorgehen ist in Abb. 7 schematisch erläutert. Dabei kann die Abdeckplatte auch erst vor Stufe c) aufgelegt werden. Der Nachweis der Bindung erfolgt mittels der geeigneten und offenbarten Verfahren.

Ein weiterer, sehr wichtiger Vorteil des erfindungsgemäßen Verfahrens besteht darin, daß das Volumen der zu untersuchenden Probe nahezu beliebig gesteigert werden kann. Selbst Probenvolumina bis zu 100 ml und mehr stellen aufgrund der unmittelbaren Verwendung der paramagnetischen Beads und der dadurch bedingten Möglichkeit des Wegfalls der Trennung von Probe und detektiertem Molekül bzw. ZZ kein Problem dar. Dabei ist eine theoretische Obergrenze für die Probengröße nur in der Verfügbarkeit der verwendeten Magnetobeads zu erkennen. Im Stand der Technik werden z.B. Bluttests ausschließlich in Volumina von bis zu 100 µl durchgeführt. Hierin ist eine Ursache für die geringe Empfindlichkeit der bisherigen Tests zu sehen, die durch vorliegende Erfindung entscheidend verbessert wird.

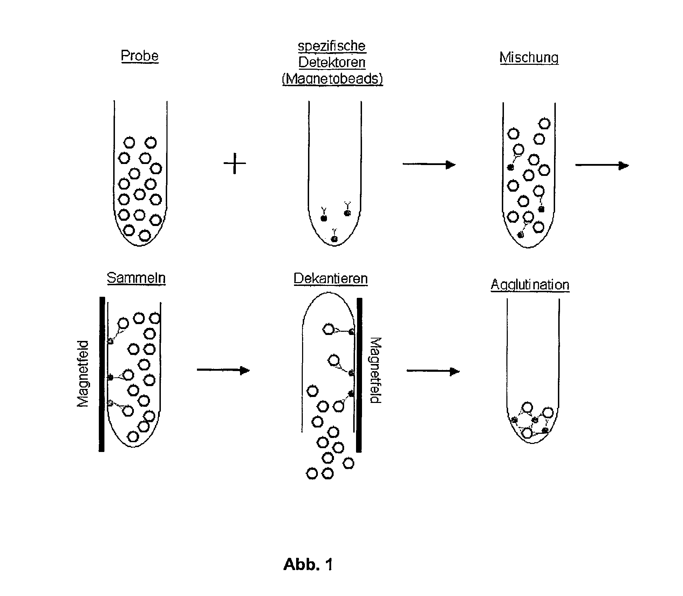

Abbildung 1 zeigt die Vorgehensweise beim erfindungsgemäßen Verfahren.

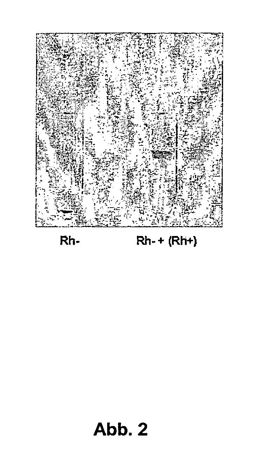

Abbildung 2 zeigt das Ergebnis der Durchführung des erfindungsgemäßen Verfahrens mit einer NaCl-Gelkarte der Fa. DiaMed, Schweiz.

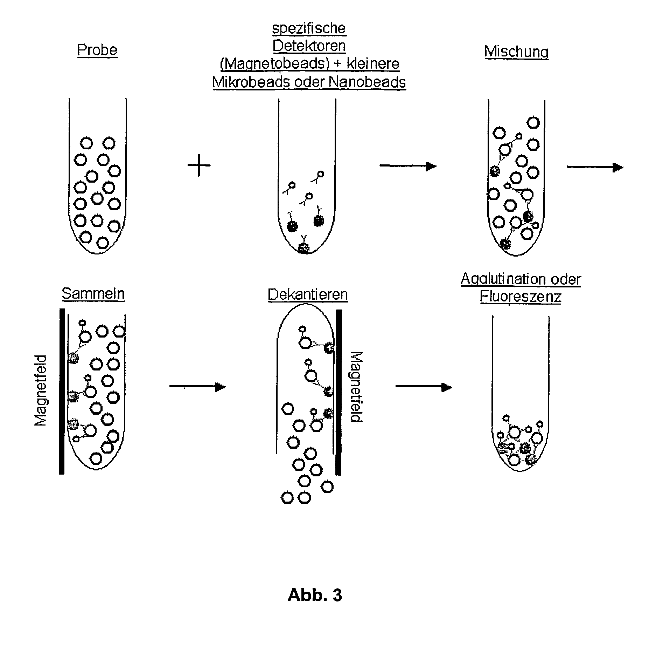

Abbildung 3 zeigt die Vorgehensweise beim erfindungsgemäßen Verfahren unter Verwendung von Agglutinationsverstärkern, die gleichzeitig mit den paramagnetischen Beads vorgelegt werden.

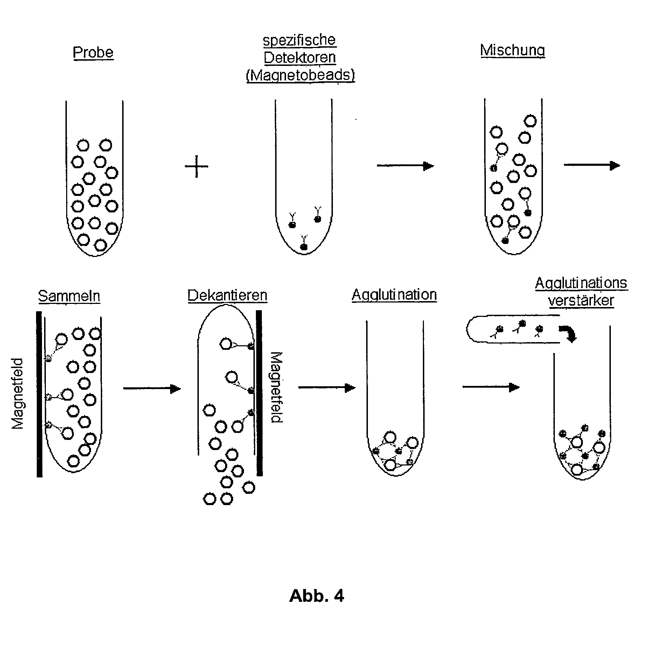

Abbildung 4 zeigt die Vorgehensweise beim erfindungsgemäßen Verfahren unter Verwendung von Agglutinationsverstärkern, die nach Isolierung und Reinigung des Biomoleküls bzw. der Zelle dazu gegeben werden.

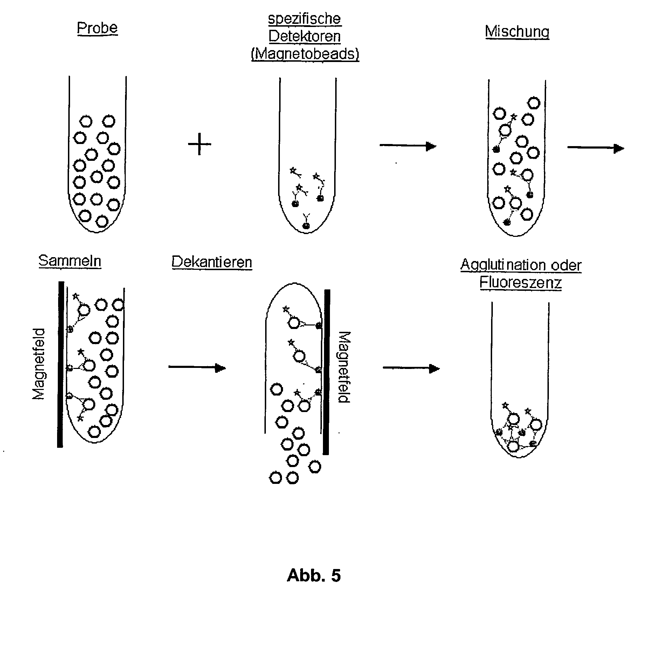

Abbildung 5 zeigt eine mögliche Vorgehensweise beim erfindungsgemäßen Verfahren unter Verwendung von Antikörpern als Agglutinationsverstärker.

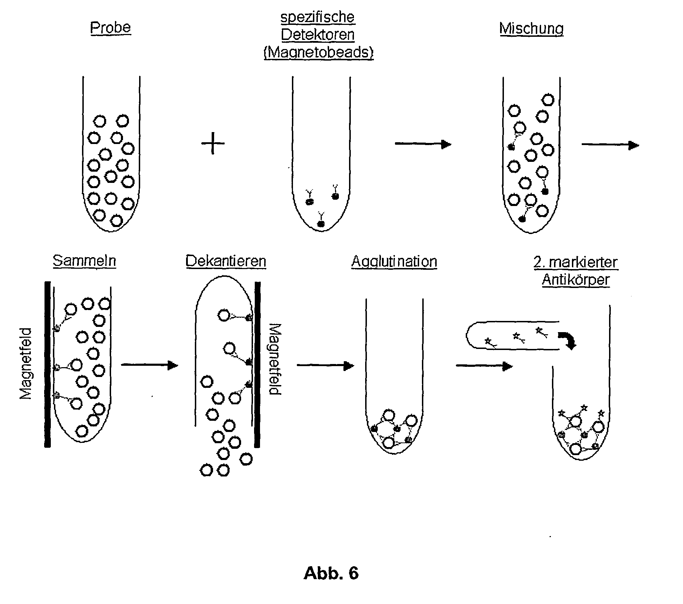

Abbildung 6 zeigt eine weitere mögliche Vorgehensweise beim erfindungsgemäßen Verfahren unter Verwendung von Antikörpern als Agglutinationsverstärker.
Abbildung 7 zeigt die Vorgehensweise beim erfindungsgemäßen Verfahren zum Nachweis von Antikörpern. Es sollen Antikörper (AK) gegen eine bestimmte Zielzelle nachgewiesen werden. Dazu wird die bestimmte Zielzelle an einem Trägermaterialohne Bindung beispielsweise durch Pipettieren in die Vertiefung einer Mikrotiterplatte vorgelegt.. An der zugehörigen Abdeckplatte sind anti-human Antikörper fest gebunden. Dann werden paramagnetische Beads, die mit einem gegen ein weiteres ZZ-spezifisches Antigen gerichteten nicht-humanem AK gekoppelt sind, dazu gegeben. Diese binden an die ZZ. Dann wird die zu untersuchende humane Probe dazugegeben, symbolisiert in Abb. 7 durch die klein gezeichneten AK, die gleichzeitig für die in der Probe eventuell vorhandenen, für die Zielzellen spezifischen AK stehen, welche - falls vorhanden - nachgewiesen werden sollen. Sind in der Probe gegen die ZZ gerichtete AK, so binden diese an die ZZ. Dann werden die auf diese Weise gebildeten Konglomerate mit Hilfe des Magneten "angehoben", so daß die Konglomerate mit den an der Unterseite der Abdeckung befindlichen anti-human AK in Kontakt treten. In den Konglomeraten sind nur dann humane AK enthalten, wenn diese als spezifische AK an die ZZ gebunden haben. Vermittelt über diese AK sowie die anti-human AK tritt nun eine spezifische Bindungsreaktion, bei insbesondere eine Agglutination auf, die direkt visuell oder auch photometrisch je nach Markierung der beteiligten Moleküle und/oder Beads beurteilt werden kann. Ein Waschschritt ist hier nicht nötig.

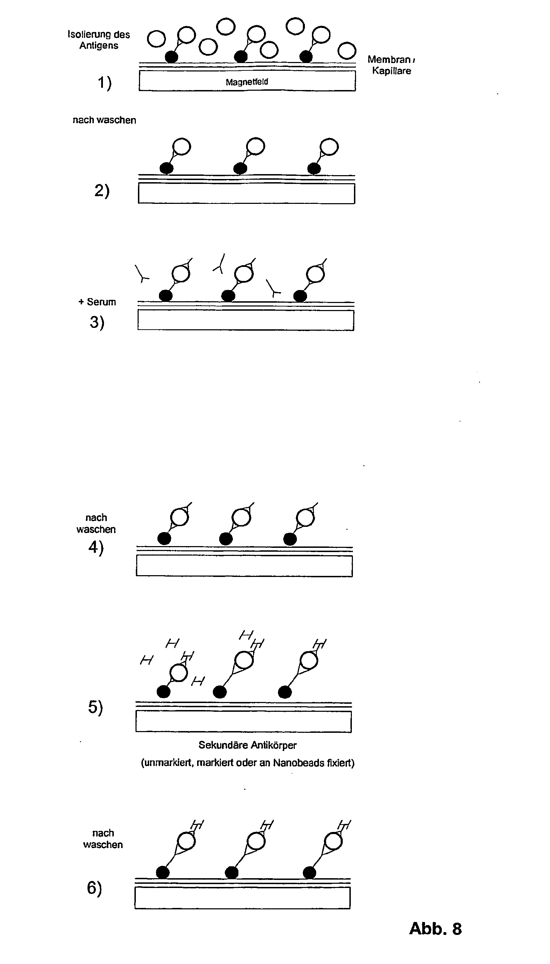

Abbildung 8 zeigt die Vorgehensweise beim erfindungsgemäßen Verfahren zum Nachweis von Antikörpern. Hier werden ebenfalls AK nachgewiesen. Zur Verstärkung der Sensitivität und zur Vermeidung unspezifischer Bindung wird hier jedoch ein Waschschritt durchgeführt. Das Vorhandensein spezifischer AK im Serum wird anhand des Vorliegens einer Agglutinationsreaktion bestimmt.

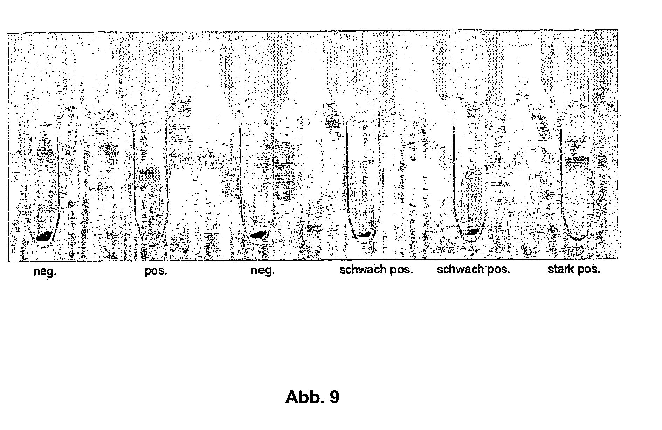

Abbildung 9 zeigt das Ergebnis aus Beispiel 1. Es ist deutlich zu erkennen, dass anti-p53-haltige Seren zur Agglutination der mit p53 beschichteten Beads führt.

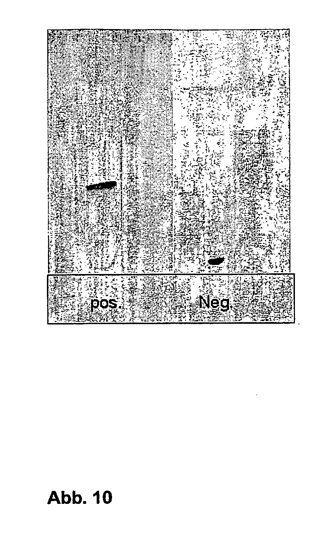

Abbildung 10 zeigt das Ergebnis aus Beispiel 2. Es ist deutlich zu erkennen, dass IgG-haltige Seren zur Agglutination der mit anti-IgG beschichteten Beads führt.

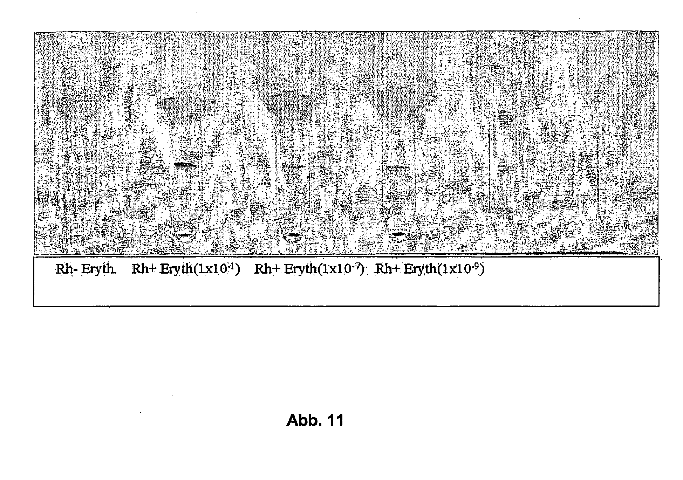

Abbildung 11 zeigt das Ergebnis aus Beispiel 3. Es ist deutlich zu erkennen, dass Rhesus positive (Rh⁺) Erythrozyten mit anti-Rh⁺ beschichteten Beads reagieren und zur Agglutination führen.

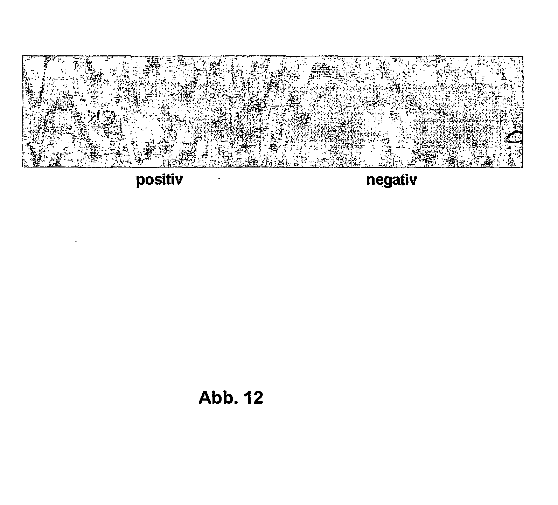

Abbildung 12 zeigt das Ergebnis aus Beispiel 4. Es ist deutlich zu erkennen, dass anti-p53 AK zur Agglutination der Beads im Röhrchen führt.

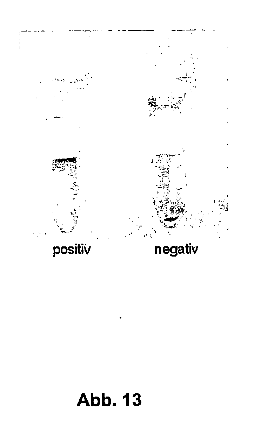

Abbildung 13 zeigt das Ergebnis aus Beispiel 5. Es ist deutlich zu erkennen, dass Autoantikörper gegen Thrombozyten (Blutplättchen) zur Agglutination der mit Plättchenantigenen beschichteten Beads führen.

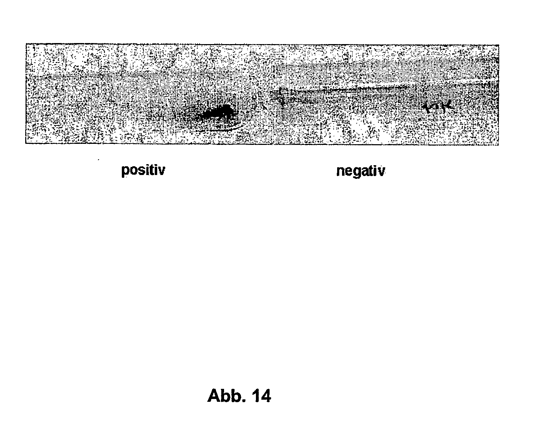

Abbildung 14 zeigt das Ergebnis aus Beispiel 6. Es ist deutlich zu erkennen, dass Rh⁺ Erythrozyten mit anti- Rh⁺ beschichteten Beads reagieren und zur Agglutination führen.

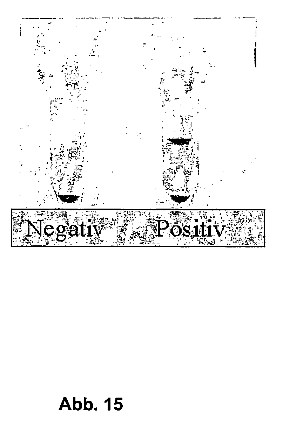

Abbildung 15 zeigt das Ergebnis aus Beispiel 7. Es ist deutlich zu erkennen, dass der AK zur Agglutination in der Gelkarte führt (AK gegen T47D).

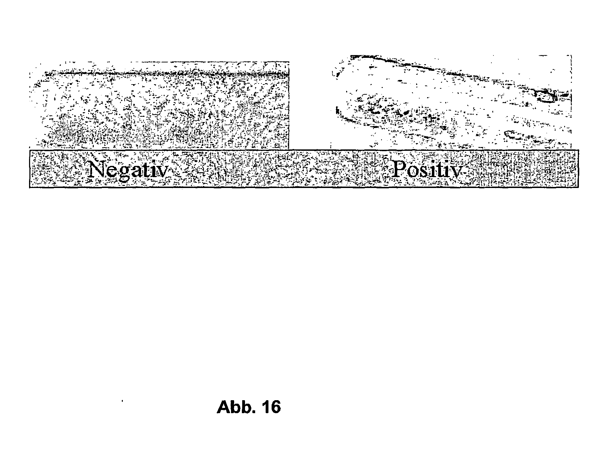

Abbildung 16 zeigt das Ergebnis aus Beispiel 7. Es ist deutlich zu erkennen, dass der AK zur Agglutination im Röhrchen führt (AK gegen T47D9.

Im Folgenden wird die Erfindung anhand von Beispielen illustriert.

### BEISPIEL 1: Nachweis von anti-P53

- Beads (Fa. Dynal, Deutschland, Magnetobeads M-270, 2.8 µm Durchmesser) wurden nach Herstellerangaben mit humanem P53 (lys320-acetyliert) (Fa. Biotrend, Köln, Deutschland) gekoppelt.
- 40 µl 0.1 % Beads (v/v) wurden mit 20 µl Serum (sowohl aus Krebspatienten sowie aus gesunden Patienten als Kontrolle) bei 37°C für 30 min. inkubiert.
- Es wurde 1 x mit Partikel-Puffer (Fa. DiaMed, Schweiz) gewaschen und in 50µl Partikel-Puffer resuspendiert.
- Die Beads wurden auf eine Gel-Karte (Fa. DiaMed, "Ziege-anti-human") 1:2 nach Herstellerangaben geschichtet und zentrifugiert.

Die verwendeten Seren 1, 2, 3 und 4 stammten von Patienten mit Verdacht auf Krebs.

Das Ergebnis ist in Abbildung 9 dargestellt. Der Nachweis von anti-P53 AK im Serum von Patienten gelang ohne Probleme.

### BEISPIEL 2: Nachweis von IgG

- Die Beads (siehe Bsp. 1) wurden mit anti-human IgG (Fa. Dianova) gekoppelt.
- Eine IgG-haltige Probe (Patientenserum) wurde mit 50µl Beads (0.1 % v/v) auf einer-NaCl-Gelkarte (Fa. DiaMed, Schweiz) bei 37°C für 15 min inkubiert und anschließend zentrifugiert. (Abb. 10).

**Ergebnis:** In Abb. 10 ist deutlich zu erkennen, dass IgG-haltige Seren zur Agglutination der mit anti-IgG beschichteten Beads führt.

### BEISPIEL 3: Detektion von Rh⁺-Zellen in einer Rh negativen (Rh⁻) Blutprobe (Gesamtmenge 3 ml).

Die Probe wurde gleichzeitig mit Magnetobeads (s. Beispiel 1) und Nanobeads (Fa. Mikropartikel, Berlin, Durchmesser 300 nm) ca. 5 min bei Raumtemperatur vermischt. Beide Sorten von Beads sind mit anti-D Antikörper (Klon BRAD3, Bristol, England) beladen. Dazu wurden 20 µg AK mit 100 µl Beads (10⁹ Beads) vermischt und nach Herstellerangaben behandelt. Die weitere Vorgehensweise entsprach den Beispielen 1 und 2.

**Ergebnis:** Die Sensitivität ist unglaublich hoch. Es kann 1 Rh⁺ Erythrozyt unter 10.000.000.000 weiteren Zellen nachgewiesen werden

### Beispiel 4: Detektion von anti-p53 im Coumbs-Röhrchen.

1) Dynalbeads (s. Beispiel 1) wurden mit humanem P53 beschichtet und mit Serum 30min bei 37°C inkubiert.
2) Es wurde 1X mit Partikelpuffer gewaschen.
3) Es wurde in 100µl Partikelpuffer resuspendiert.
4) Dazu wurden 10µl Nanobeads (100nm Durchmesser, Kanninchenanti-human) gegeben und 30sec bei 300 g in der Coumbs-Zentrifuge (Hersteller: Immuncore) zentrifugiert (Abb. 12).

**Ergebnis:** Es ist deutlich zu erkennen, dass anti-p53 AK zur Agglutination der Beads im Röhrchen führt.

### Beispiel 5: Nachweis von thrombozytären Antikörpern mithilfe einer Gelkarte:

1) Dynalbeads (s. Beispiel 1) wurden mit AK anti-CD41 bzw. anti-CD49b (monoklonale Antikörper gegen Thrombozytenantigene) beschichtet und mit Thrombozyten-Solubilisat (40 µl Konzentrat) und 20 µl Serum 30min bei 37°C inkubiert.
2) Es wurde 1X mit Partikel puffer gewaschen.
3) Es wurde in 50µl Partikelpuffer resuspendiert und auf eine Ziege-anti-human Gelkarte (Dynal) 1:4 nach Herstellerangaben aufgetragen(Abb. 13). Das weitere Vorgehen war wie in den Beispielen 1 bzw. 2.

**Ergebnis:** Es ist deutlich zu erkennen, dass Autoantikörper gegen Thrombozyten (Blutplättchen) zur Agglutination der mit Plättchenantigenen beschichteten Beads führen.

### Beispiel 6: Nachweis von RH⁺-Zellen im Coumbs-Röhrchen:

1) 100µl Dynalbeads (s. Beispiel 1) wurden mit Anti-D (Beispiel 3) beschichtet und mit 10µl mit Anti-D beschichteten Nanobeads (s. Beispiel 4, 100 µm Durchmesser) 10 min. inkubiert.
2) Die Beads wurden mit dem Magneten aus dem Blut isoliert
3) Es wurde 1X mit NaCl gewaschen
4) Es wurde in 100µl NaCl resuspendiert und 30 sec bei 300 g in der Coumbs Zentrifuge (Immuncore) zentrifugiert (Abb. 14).

**Ergebnis:** Es ist deutlich zu erkennen, dass Rh⁺ Erythrozyten mit anti-Rh⁺ (anti-D) beschichteten Beads reagieren und zur Agglutination führen.

### Beispiel 7: Detektion von epithelialen Tumorzellen (Zelllinie T47D) in 2ml EDTA-Blut mit dem Klon 5E11 sowohl mit Hilfe einer Gelkarte (DiaMed) als auch direkt in Blut:

1) 10µl des 1:5 verdünnten und nach Standardverfahren biotinylierten Antikörpers (Klon 5E11, Dianova EP4) wurden mit 10µl Streptavidin-Beads (Dynal M-280) 0,5 h im Inkubator bei 37°C gekoppelt.
2) Es wird 2x mit 100 µl Partikelpuffer gewaschen.
3) Es wird in 100 µl Partikelpuffer aufgenommen und in 2ml EDTA-Blut mit T47D-Zellen (als Positivprobe) bzw. in 2ml EDTA-Blut ohne T47D (Negativprobe) gegeben.
4) 15 min. Inkubation auf dem Rotor.
5) Dann wurde 10 min. auf Magnet separiert.
6) Die Beads wurden in 50µl Partikelpuffer aufgenommen (bei der PositivProbe bilden sich bei diesem Schritt Agglutinate ) und (a) auf eine Karte (Dynal) (Abb. 15) gegeben und 10 min. in der Kartenzentrifuge zentrifugiert sowie (b) im Röhrchen nachgewiesen (Abb. 16)

**Ergebnis:** Es ist deutlich zu erkennen, dass der AK zur Agglutination in der Gelkarte führt (AK gegen T47D, Abb. 15). Es ist auch deutlich zu erkennen, dass der AK zur Agglutination im Röhrchen führt (AK gegen T47D9, Abb. 16).

## Patentansprüche

1. Verfahren zum Nachweis von ausgewählten Zellen (ZZ) oder Biomolekülen in einer Probe, **dadurch gekennzeichnet dass** man
a) paramagnetische Mikrobeads mit einem gegen ein Merkmal der Biomoleküle oder Zellen gerichteten spezifischen Detektionsmolekül beschichtet,
b) die Probe mit den beschichteten Mikrobeads in Kontakt bringt,
c) die Probe von den nun beladenen Mikrobeads mit Hilfe eines anzulegenden Magneten entfernt,
d) die beladenen und von der Probe entfernten Mikrobeads in Suspension bringt,
e) die in d) erhaltenen Mikrobeads-Suspension im Gelkartensystem untersucht, und
f) anhand des Vorliegens einer Agglutinierung zwischen den Biomolekülen und/oder den ZZ und den beschichteten Mikrobeads direkt das Vorhandensein des nachzuweisenden Biomoleküls bzw. der nachzuweisenden Zelle visuell oder photometrisch bestimmt.

2. Verfahren nach Anspruch 1, **dadurch gekennzeichnet, dass** die Mikrobeads eine Größe zwischen 0,1 und 5 µm, bevorzugt 2,5 und 3 µm aufweisen und bevorzugt farbig oder mit Europium markiert sind.

3. Verfahren nach einem der vorstehenden Ansprüche, **dadurch gekennzeichnet, dass** zusätzlich im Reaktionsgemisch mit einem spezifischen, gegen die nachzuweisende Zelle oder das nachzuweisende Biomolekül gerichtetes Detektionsmolekül beschichtete, bevorzugt farbige oder mit Europium markierte Nanobeads als Agglutinationsverstärker vorgelegt werden.

4. Verfahren nach Anspruch 3, **dadurch gekennzeichnet, dass** die Nanobeads eine Größe von 50 nm bis 0,5 µm, bevorzugt etwa 100 nm aufweisen.

5. Verfahren nach einem der vorstehenden Ansprüche, **dadurch gekennzeichnet, dass** zusätzlich spezifische, gegen die nachzuweisende Zelle oder das nachzuweisende Biomolekül gerichtete, bevorzugt markierte Zweitantikörper als Agglutinationsverstärker mitgeführt werden.

6. Verfahren nach einem der vorstehenden Ansprüche, **dadurch gekennzeichnet, dass** zwischen Stufe (c) und (d) ein Waschvorgang oder mehrere Waschvorgänge durchgeführt werden.

7. Verfahren nach einem der vorstehenden Ansprüche, **dadurch gekennzeichnet, dass** für die Schritte e) bis g) ein Coombs-Röhrchen statt einer Gelkarte verwendet wird.

8. Verfahren nach einem der vorstehenden Ansprüche, **dadurch gekennzeichnet , dass** die Nanopartikel und/oder Mikrobeads farbig sind.

9. Verfahren nach Anspruch 8, **dadurch gekennzeichnet, dass** die Beurteilung des Vorliegens einer spezifischen Reaktion photometrisch durchgeführt wird.

10. Verfahren nach einem der vorstehenden Ansprüche, **dadurch gekennzeichnet, dass** die Nanopartikel und/oder Mikrobeads mit Europium markiert sind, und der Nachweis der Agglutinate durch Fluoreszenzmessung durchgeführt wird.

11. Verfahren nach einem der vorstehenden Ansprüche, **dadurch gekennzeichnet, dass** die Biomoleküle Antikörper und die Detektionsmoleküle die entsprechenden Antigene sind.

12. Verfahren nach Anspruch 10, **dadurch gekennzeichnet, dass** die Antigene in nativer Form, bevorzugt in Form eines bestimmten Zelltyps, vorliegen.

## Claims

1. Method of detecting selected cells (ZZ) or biomolecules in a sample,
**characterised in that**
a) paramagnetic microbeads are coated with a specific detection molecule which is directed against a marker of the biomolecules or cells,
b) the sample is brought into contact with the coated microbeads,
c) the sample is removed from the now loaded microbeads by means of a magnet which has to be applied,
d) the loaded microbeads which have been removed from the sample are placed in suspension,
e) the microbead suspension obtained in d) is examined by the gel card system, and
f) the presence of the biomolecule to be detected or the cell to be detected is directly established, visually or photometrically, from the presence of agglutination between the biomolecules and/or the selected cells (ZZ) and the coated microbeads.

2. Method according to claim 1, **characterised in that** microbeads are of a size of between 0.1 and 5 µm, and preferably between 2.5 and 3 µm, and are marked, preferably in colour or with europium.

3. Method according to either of the foregoing claims, **characterised in that** there are provided in addition in the reaction mixture, as agglutination enhancers, nanobeads which are coated with a specific detection molecule which is directed against the cell to be detected or biomolecule to be detected and which are marked, preferably in colour or with europium.

4. Method according to claim 3, **characterised in that** the nanobeads are of a size of from 50 nm to 0.5 µm, and preferably of approximately 100 nm.

5. Method according to one of the foregoing claims, **characterised in that** included in addition, as agglutination enhancers, are specific second antibodies which are directed against the cell to be detected or the biomolecule to be detected and which are preferably marked.

6. Method according to one of the foregoing claims, **characterised in that** a washing operation or a plurality of washing operations are performed between steps (c) and (d).

7. Method according to one of the foregoing claims, **characterised in that** a Coombs tube rather than a gel card is used for steps (e) to (g).

8. Method according to one of the foregoing claims, **characterised in that** the nanoparticles and/or nanobeads are coloured.

9. Method according to claim 8, **characterised in that** the assessment of the presence of a specific reaction is performed photometrically.

10. Method according to one of the foregoing claims, **characterised in that** the nanoparticles and/or microbeads are marked with europium and the detection of the agglutinates is performed by the measurement of fluorescence.

11. Method according to one of the foregoing claims, **characterised in that** the biomolecules are antibodies and the detection molecules are the corresponding antigens.

12. Method according to claim 10, **characterised in that** the antigens are present in native form and preferably in the form of a given cell type.

## Revendications

1. Procédé de détection de cellules (ZZ) ou de biomolécules sélectionnées dans un échantillon, **caractérisé en ce que**
a) on revêt des microbilles paramagnétiques avec une molécule de détection spécifique dirigée contre une caractéristique des biomolécules ou des cellules,
b) on met l'échantillon en contact avec les microbilles revêtues,
c) on sépare l'échantillon des microbilles maintenant chargées à l'aide d'un aimant à leur appliquer,
d) on met les microbilles chargées et séparées de l'échantillon en suspension,
e) on examine la suspension de microbilles obtenue dans d) dans un système de carte gel, et
f) grâce à la présence d'une agglutination entre les biomolécules et/ou les ZZ et les microbilles revêtues, on détecte directement, visuellement ou par photométrie, la présence de la biomolécule à détecter ou de la cellule à détecter.

2. Procédé selon la revendication 1, **caractérisé en ce que** les microbilles ont une taille entre 0,1 et 5 µm, de préférence entre 2,5 et µm et sont de préférence colorées ou marquées avec de l'europium.

3. Procédé selon l'une des revendications précédentes, **caractérisé en ce que** le mélange réactionnel contient en outre, en tant qu'agent renforçant l'agglutination, des nanobilles revêtues d'une molécule de détection spécifique dirigée contre la cellule à détecter ou la biomolécule à détecter, de préférence colorées ou marquées avec de l'europium.

4. Procédé selon la revendication 3, **caractérisé en ce que** les nanobilles ont une taille de 50 nm à 0,5 µm, de préférence d'environ 100 nm.

5. Procédé selon l'une des revendications précédentes, **caractérisé en ce que** l'on ajoute en outre, comme agents renforçant l'agglutination, des deuxièmes anticorps spécifiques dirigés contre la cellule à détecter ou la biomolécule à détecter, de préférence marqués.

6. Procédé selon l'une des revendications précédentes, **caractérisé en ce que**, entre les étapes (c) et (d), on effectue une opération de lavage ou plusieurs opérations de lavage.

7. Procédé selon l'une des revendications précédentes, **caractérisé en ce que**, pour les étapes e) à g), on utilise un tube Coombs à la place d'une carte gel.

8. Procédé selon l'une des revendications précédentes, **caractérisé en ce que** les nanoparticules et/ou les microbilles sont colorées.

9. Procédé selon la revendication 8, **caractérisé en ce que** l'évaluation de la présence d'une réaction spécifique s'effectue par photométrie.

10. Procédé selon l'une des revendications précédentes, **caractérisé en ce que** les nanoparticules et/ou les microbilles sont marquées avec de l'europium, et la détection des agglutinats s'effectue par mesure de la fluorescence.

11. Procédé selon l'une des revendications précédentes, **caractérisé en ce que** les biomolécules sont des anticorps et les molécules de détection les antigènes correspondants.

12. Procédé selon la revendication 10, **caractérisé en ce que** les antigènes se trouvent sous forme native, de préférence sous forme d'un certain type de cellule.
